# EUROPEAN PATENT SPECIFICATION

(11) **EP 2 525 744 B1**
(45) Date of publication and mention of the grant of the patent: **19.11.2014**
(21) Application number: 10798700.0
(22) Date of filing: 28.12.2010
(51) Int. Cl.: A61F 2/42, A61F 2/00, A61F 2/30

(54) **RESURFACING IMPLANT**
OBERFLÄCHENERSATZIMPLANTAT
IMPLANT DE RESURFAÇAGE

(30) Priority: 22.01.2010 US 692200
(43) Date of publication of application: 28.11.2012
(73) Proprietor: OSTEOMED LLC, Addison, TX 75001 (US)
(72) Inventor: BECKENDORF, Brandon, G., Arlington TX 76002 (US); HERNANDEZ, Geronimo, Fort Worth TX 76119 (US)
(74) Representative: Lawrence, John
(86) International application number: PCT/US2010/062216
(87) International publication number: WO 2011/090711

(56) References cited:
- EP-A1- 1 136 047
- DE-A1- 10 358 307
- DE-U1-202007 009 620
- US-A- 2 622 592
- US-A1- 2007 156 250

## Description

### TECHNICAL FIELD

The present disclosure generally relates to orthopedic devices, and more particularly, to a resurfacing implant.

### BACKGROUND

When performing certain medical procedures, such as repairing a joint that has been damaged due to disease or trauma, a surgeon may need to resurface one of the bones of the joint in order to restore the joint's ability to articulate. One way to achieve this objective is to insert an implant into the bone to restore the articulating surface of that bone. Examples of previous implant designs can be found in DE 202007009620, which forms the basis for the preamble of claim 1, and in US 2622592, EP 1136047, DE 10358307, and US 2007/156250.

### SUMMARY

In particular embodiments, an implant for use on a joint-facing end of a bone includes a cap having convex outer surface overlying a concave inner surface and a stem protruding from the inner surface. Depending upon design, an edge of the cap may surround the stem and overhang a portion of the stem leading up to the inner surface. The stem may further include a plurality of raised ridges parallel to its length that begin near a tip of the stem and extend onto the portion of the stem overhung by the edge.

The outer surface may comprise a vertical axis and a horizontal axis. The vertical axis may be situated perpendicular to the horizontal axis. Depending upon design, a degree of curvature of the outer surface along the vertical axis may be greater than a degree of curvature of the outer surface along the horizontal axis.

In particular embodiments, the inner surface may be generally spherical and the outer surface may be contoured to mimic the natural geometry of a surface of a metatarsal bone.

In certain constructs, the outer surface may be separated from the inner surface by, at most, one millimeter of material.

As to the stem, in accordance with the invention, it includes a generally smooth cylindrical portion disposed between the tip and the plurality of raised ridges.

Depending upon design, at least one of the plurality of raised ridges may include at least one notch adapted to prevent the stem from backing out of a bone once the stem is implanted into the bone. For example, the at least one notch may be a hook-shaped recession in the at least one ridge.

In particular embodiments, the plurality of raised ridges may traverse a majority of the portion of the stem overhung by the edge.

In certain constructs, a height of at least one of the plurality of raised ridges may generally increase relative to a central axis of the stem as the at least one raised ridge approaches the inner surface of the cap.

The inner surface of the cap and the stem may also include a porous coating and a biologic coating to promote bone growth.

A method for using an implant on a joint-facing end of a bone which is not falling within the scope of the invention may include a number of steps. For example that method may include the step of providing an implant having a cap with a convex outer surface overlying a concave inner surface and a stem protruding from the inner surface such that an edge of the cap surrounds the stem and overhangs a portion of the stem leading up to the inner surface. The stem may include a plurality of raised ridges parallel to its length that begin near a tip of the stem and extend onto the portion of the stem overhung by the edge. The method may further include the steps of creating an elongated pathway into an end of a metatarsal bone for the stem, inserting the stem into the pathway, and pressing the stem into the pathway until the inner surface of the cap comes to bear on the end of the metatarsal bone.

In particular embodiments, an implant for use on a joint-facing end of a bone, may include a cap having convex outer surface overlying a concave inner surface and a stem protruding from the inner surface. The outer surface has a vertical axis and a horizontal axis, wherein the vertical axis is situated perpendicular to the horizontal axis. Depending upon design, a degree of curvature of the outer surface along the vertical axis may be greater than a degree of curvature of the outer surface along the horizontal axis.

Particular embodiments of the present disclosure may provide a number of technical advantages, including for example, promoting natural articulation of a repaired joint, reducing the amount of bone resection needed to implant the implant onto a bone, and providing a surgeon with relatively simple procedure for implanting the implant into a bone. Other technical advantages of the present disclosure will be readily apparent to one skilled in the art from the following figures, descriptions, and claims. Moreover, while specific advantages have been enumerated above, various embodiments may include all, some, or none of the enumerated advantages.

### BRIEF DESCRIPTION OF THE DRAWINGS

For a more complete understanding of the present disclosure and its advantages, reference is now made to the following descriptions, taken in conjunction with the accompanying drawings, in which:
FIGURE 1 illustrates a failed metatarso-phalangeal joint in the big toe of a human foot;
FIGURE 2 illustrates a resurfacing implant being used to repair the failed metatarso-phalangeal joint of FIGURE 1 according to an example embodiment of the present disclosure; and
FIGURES 3 and 4 illustrates a more detailed isometric view of the resurfacing implant of FIGURE 2.

### DETAILED DESCRIPTION OF EXAMPLE EMBODIMENTS

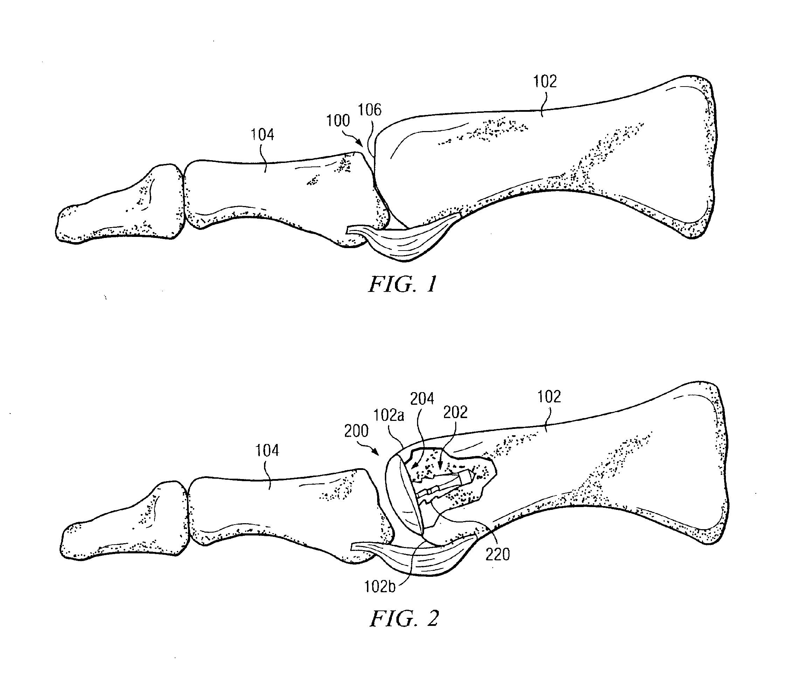

As shown in FIGURE 1, the first metatarso-phalangeal joint 100 is a joint between a metatarsal bone 102 of the foot and the proximal phalanx bone 104 of the big toe. Certain diseases or trauma may injure metatarso-phalangeal joint 100, causing permanent stiffness (known as hallux rigidus), limited flexibility (known as hallux limitus), or misalignment (known as hallux valgus). Patients suffering from any one of those conditions may have difficulty running or walking and may experience pain while engaging in either of those activities, or in other activities that put a load on metatarso-phalangeal joint 100.

Mild cases may often be treated using conservative measures such as taking antiinflammatory medications to reduce inflammation in joint 100, wearing specialized foot ware designed to inhibit flexion of joint 100, and/or avoiding activities that challenge joint 100. However, in more severe cases, such as illustrated in FIGURE 1 where joint 100 has completely degraded, conservative measures may be ineffective, and surgery may be required.

One procedure for reconstructing joint 100 involves fusing the bones of joint 100 together using screws and/or plates. More particularly, a fusion procedure may involve reducing the opposing faces of the bones of joint 100 to a bleeding bone bed, approximating the bones with one another, and screwing the bones together to promote fusion. In some cases, a plate may be screwed across the joint to provide additional stability. However, this option eliminates joint 100's ability to flex, places more stress on the surrounding joints, and may not be appropriate for patients with active life styles.

Another option for surgically repairing joint 100 involves replacing it with an artificial joint. More particularly, a full joint replacement procedure may involve reducing the opposing faces of the bones of joint 100 to a bleeding bone bed, inserting cup-like implant into one bone, inserting a ball-like implant into the other bone, and fitting the ball-like implant into the cup-like implant to create the artificial joint. However, due to the ball- and-socket nature of the assembly, this option may result in unnatural articulation of joint 100, allowing lateral bending of the big toe, for example. Also, in many cases, it may be unnecessary to replace both sides of joint 100, especially if only one of the bones of the joint is damaged.

Yet another, less intensive, procedure for surgically repairing a damaged joint involves replacing only one side of the joint with an artificial implant. As compared to either of the two surgical procedures discussed above, this option may result in a more natural range of motion in the joint and a speedier recovery time for the patient. When this type of procedure is performed on the metatarso-phalangeal joint, the head of the metatarsal bone or the base of the phalangeal bone may be replaced with an implant that replaces the articulating surface of the bone on which it is implanted.

FIGURE 2 illustrates an example embodiment of a resurfacing implant 200 being used to repair the damaged metatarso-phalangeal joint 100 of FIGURE 1. As shown in FIGURE 2, resurfacing implant 200 has been attached to the joint-facing end of metatarsal bone 102. Although the present disclosure portrays resurfacing implant 200 being used on the first metatarsal bone in the first metatarso-phalangeal joint 100 of the big toe, one of ordinary skill in the art will appreciate that that specific example is presented for the sake of explanatory clarification and will further appreciate that resurfacing implant 200 may be adapted equally as well for use on any suitable bone in any suitable joint of the body. For example, various embodiments of resurfacing implant 200 may be adapted in size and configuration for use in metacarpal joints, carpal joints, tarsal joints, and spinal joints.

A surgeon may use resurfacing implant 200 to repair joint 100 according to the following example surgical procedure. To begin the procedure, the surgeon may create an incision over joint 100 to expose metatarsal bone 102. After exposing metatarsal bone 102, the surgeon may measure the head 106 (see Figure 1) of the joint-facing end of metatarsal bone 102 to determine the appropriate size for resurfacing implant 200. Typically, resurfacing implant 200 should be sized to replace the entire articulating surface of the joint-facing end of metatarsal bone 102.

After selecting a resurfacing implant 200 of the appropriate size, the surgeon may sculpt metatarsal head 106 to fit snugly into the concave under side of resurfacing implant 200. For example, the surgeon may shave off a portion of metatarsal head 106 so that it fits snugly into the concave underside of resurfacing implant 200. After the sculpturing process, the surgeon may create a pathway into metatarsal bone 102 to accommodate the stem 202 of resurfacing implant 200. To create that pathway, the surgeon may drill a pilot hole into bone 102 along the desired trajectory for stem 202. Typically, the pathway will be coaxial with the central axis of metatarsal bone 102. After creating the pilot hole, the surgeon may use a device such as a broach to create a number of indentions around the pilot hole to accommodate raised ridges 220 that may be present on stem 202.

Once the surgeon has finished that process, the cross-sectional shape of the pathway may generally correspond to the cross-sectional shape of stem 202, but may be slightly smaller in dimension than stem 202 to ensure a tight fit between stem 202 and the pathway. For example, both the pathway and stem 202 may have a generally cruciform-like cross-sectional shape; however, the cruciform shaped pathway in bone 102 may be smaller than the cross-section of stem 202. Next, the surgeon may insert stem 202 into the pathway such that raised ridges 220 align with their respective indentions in the pathway and press resurfacing implant 200 into metatarsal bone 102 until the underside of cap 204 of resurfacing implant 200 comes to bear on metatarsal head 106. In certain procedures, the surgeon may optionally apply bone cement to stem 202 or the pathway prior to inserting stem 202 into the pathway to help bond stem 202 to bone 102. After ensuring that resurfacing implant 200 fits snugly onto metatarsal head 106, the surgeon may realign joint 100 and close the incision, leaving the patient to heal.

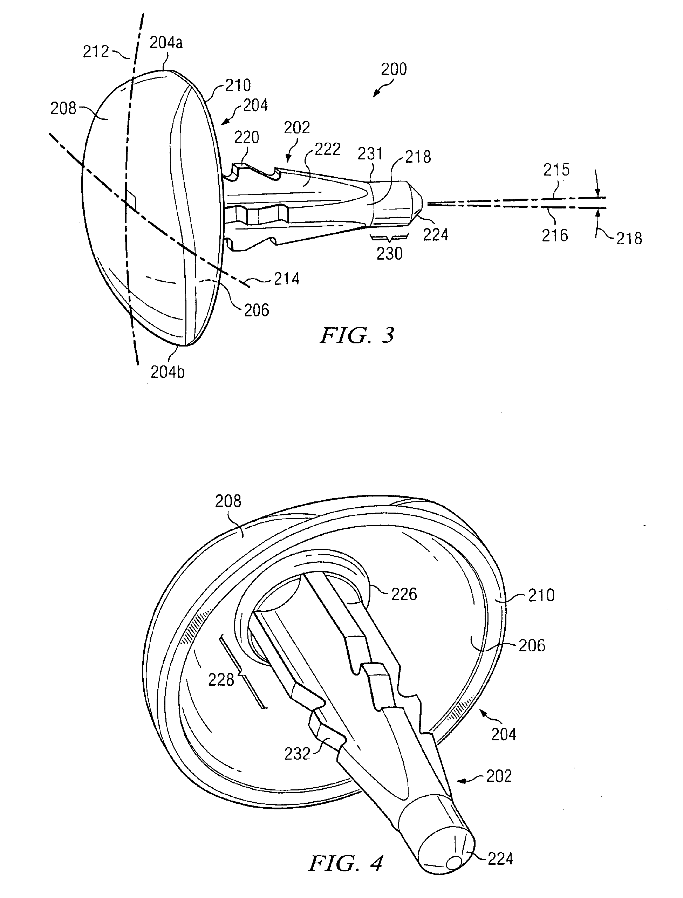

FIGURES 3 and 4 illustrate more detailed isometric views of resurfacing implant 200. Resurfacing implant 200 generally includes cap 204 and stem 202. Cap 204 is typically fused to stem 202 in a single rigid construction. However, it is also possible that stem 202 could be removeably coupled to cap 204, such as for example by a threaded engagement to enable different sized caps 204 to be attached to stem 202.

Cap 204 may be any rigid, generally dome-shaped, fixture capable of covering metatarsal head 106. Cap 204 generally includes a convex outer surface 208 that overlies a concave inner surface 206. Outer surface 208 and inner surface 206 may meet to form a generally circular edge 210 of cap 204. Depending upon the size and shape of cap 204, edge 210 may overhang a portion 228 of stem 202 (see FIGURE 4). That is, the overhung portion 228 of stem 202 may be defined as the portion of stem 202 residing between the plane of edge 210 and the point of connection 226 of stem 202 to inner surface 206.

In a typical construction, the thickness of cap 204 may be less than or equal to one millimeter ("1 mm") at any point other than where cap 204 is connected to stem 202. Limiting the thickness of cap 204 to 1 mm or less, may enable a surgeon to implant resurfacing implant 200 onto metatarsal head 106 without substantially shortening the length of metatarsal bone 102. For example, in the procedure described above, the surgeon may only need to remove 1 mm of bone length from metatarsal bone 102 in order to make room for resurfacing implant 200. Consequently, metatarsal bone 102 may be left largely intact after implantation of resurfacing implant 200, leaving adequate bone mass for a more intensive procedure, such as a total joint replacement, later in time, if needed.

To facilitate proper placement of resurfacing implant 200 onto metatarsal head 106, inner surface 206 may be configured to have a regular shape that will fit easily and snugly over the sculptured end of metatarsal head 106. As an example and not by way of limitation, inner surface 206 may be a spherically-shaped bowl. During the sculpturing process described above, the surgeon may sculpt metatarsal head 106 into a corresponding dome that fits snugly into inner surface 206 and rests flush against inner surface 206, once cap 204 is placed over metatarsal head 106. This may eliminate any pressure points between metatarsal head 106 and inner surface 206 and also enable the surgeon to feel when metatarsal cap 204 is properly seated on metatarsal head 106. One of ordinary skill in the art will appreciate that the above-described embodiment of inner surface 206 was presented for the sake of explanatory clarity and will further appreciate that inner surface 206 could have other geometries (e.g., cylindrical or cubical geometries) that would work equally as well, provided that metatarsal head 106 is similarly tailored to fit into those geometries.

Outer surface 208 generally includes a top or "dorsal" side 204a intended to align with the dorsal side 102a of metatarsal bone 102 and a bottom or "plantar" side 204b intended to align with the plantar side 102b of metatarsal bone 102. Various features of outer surface 208 may be described with respect to its vertical axis 212, which runs from dorsal side 204a to plantar side 204b and its horizontal axis 214, which is situated perpendicular to vertical axis 212.

In particular embodiments, the geometry of outer surface 208 may be tailored to accomplish certain goals such as to facilitate natural joint movement, to avoid impinging on the sesamoid bones or altering sesamoid articulation, and to avoid interfering with the normal balance of the flexor-extensor tendons, plantar plate, or abductor-abductor mechanisms. As an example and not by way of limitation, the degree of curvature of outer surface 208 along vertical axis 212 may be greater on dorsal side 204a than on plantar side 204b. That characteristic may promote dorsi-flexion, which enables the toe to naturally bend upwards during activities such as running or walking, while inhibiting plantar-flexion, which may result in unnatural downward movement of the toe. As another example and not by way of limitation, the average degree of curvature along horizontal axis 214 may be less than the average degree of curvature along vertical axis 212. That characteristic may promote natural dorsal-plantar movement of the toe, while inhibiting unnatural medial-lateral movement of the toe. By combining various of those characteristics, outer surface 208 may be tailored to mimic the natural geometry metatarsal head 106. In particular embodiments, outer surface 208 may be symmetric about vertical axis 212 to facilitate use on either right-side or left-side joints.

Stem 202 may be any generally elongate protrusion extending from inner surface 206, and capable of being implanted in metatarsal bone 102 to secure resurfacing implant 200 to metatarsal bone 102. As an example and not by way of limitation, stem 202 may be a rigid pin secured to the center of inner surface 206. In particular embodiments, stem 202 may range in length from 15 mm to 19 mm. Stem 202 may further be defined by a solid central shaft 218 surrounded by a number of raised ridges 220. Each raised ridge 220 may be separated from the next by a groove 222. Stem 202 may further include, at one end, a pointed tip 224 and at the other end, point of connection 226. Stem 202 also includes a smooth cylindrical portion 230 disposed immediately adjacent to tip 224. Smooth cylindrical portion 230 may help guide stem 202 into the pathway created in bone 102 by the surgeon in the procedure described above.

Depending upon design, the longitudinal axis 215 of stem 202 may be slightly offset from the central axis 216 of outer surface 208 in order to bias cap 204 in a particular orientation on metatarsal head 106. As an example and not by way of limitation, longitudinal axis 215 may diverge from central axis 216 by an angle 218 of approximately six degrees toward dorsal side 204a. Consequently, when stem 202 is press-fitted into bone 102, cap 204 may settle onto metatarsal head 106 biased toward dorsal side 102a. That biasing may promote dorsi-flexion of joint 100 during activities such as walking.

As mentioned above, in particular embodiments, stem 202 may include a plurality of raised ridges 220. Each raised ridge 220 may be a generally fin-like crest rising out of central shaft 218 in a plane parallel to longitudinal axis 215. Although raised ridges 202 may extend over any portion of stem 202, in one design, raised ridges 220 begin at the trailing edge 231 of smooth cylindrical portion 230 and extend along the length of stem 202 to point of connection 226. Raised ridges 220 may also increase in height relative to longitudinal axis 215 as they extend along the length of stem 202. For example, raised ridges may be shortest near trailing edge 231 where they begin and tallest near point of connection 226 where they end. In particular embodiments, raised ridges 220 may be positioned around central shaft 218 at regular intervals such that an equal amount of space separates each raised ridge 220 from the next. As an example and not by way of limitation, stem 202 may include four raised ridges 220 disposed at right angels to one another. In that embodiment, stem 202 would have a generally cruciform cross-sectional shape.

Raised ridges 220 may serve several functions. For example, they may help guide stem 202 into the pathway created in bone 102 by the surgeon using the procedure described above; they may prevent resurfacing implant 200 from rotating within bone 102 once it is implanted, and the portion of raised ridges 220 disposed on overhung portion 228 may squeeze bone 102 against inner surface 206 to help ensure a tight pressure fit between metatarsal head 106 and cap 204. The portion of raised ridges 220 disposed on overhung portion 228 may also provide the sturdiest point of attachment between bone 102 and stem 202 since bone 102 is typically densest near metatarsal head 106. Although raised ridges 220 have been illustrated and described as being uniform in size and shape to one another, it is also possible that that one or more raised ridges could vary in height or length from the others.

In particular embodiments, one or more raised ridges 220 may include one or more notches 232. Each notch 232 may be a recession in raised ridge 220 configured to inhibit stem 202 from backing out of bone 102 once stem 202 is inserted into bone 102. For example, a notch 232 may be a generally fishhook-shaped recession in a raised ridge 220, adapted at the hook-end to bite into bone 102 when stem 202 is pulled out. Notches 232 of other shapes would also suffice so long as they create teeth or serrations in raised ridges 220 that would impede stem 202 from backing out of bone 102.

Resurfacing implant 200 may be formed from any material or combination of materials suitable for forming medical implants. Such materials may have high strength-to-weight ratios and may be inert to human body fluids. As an example and not by way of limitation, resurfacing implant 200 may be formed from a cobalt chromium alloy (ASTM F-1537). That alloy may provide several benefits as a material for resurfacing implant 200 such as being relatively lightweight, providing adequate strength for withstanding forces typically experienced by a joint implant, and being visible in radiographs of the implant region. Also, some or all of the portions of resurfacing implant 200 intended to contact bone 102 (e.g., stem 202 inner surface 206) may be coated with a porous coating such as titanium plasma and a biologic compound such as Hydroxyapatite to promote bone growth.

## Claims

1. An implant (200) for use on a joint-facing end of a bone comprising:
a cap (204) having convex outer surface (208) overlying a concave inner surface (206); and
a stem (202) protruding from the inner surface (206) such that an edge (210) of the inner surface (206) surrounds the stem (202) and overhangs a portion (228) of the stem (202) leading up to the inner surface (206), the stem (202) including a plurality of raised ridges (220) parallel to its length that begin near a tip (224) of the stem (202) and extend onto the portion (228) of the stem (202) overhung by the edge (210), **characterised in that** the stem (202) includes a smooth cylindrical portion (230) disposed between the tip (224) and the plurality of raised ridges (220).

2. The implant (200) of Claim 1, wherein:
the outer surface (208) comprises a vertical axis (212) and a horizontal axis (214), the vertical axis (212) situated perpendicular to the horizontal axis (214); and
a degree of curvature of the outer surface (208) along the vertical axis (212) is greater than a degree of curvature of the outer surface (208) along the horizontal axis (214).

3. The implant (200) of Claim 1, wherein the inner surface (206) is generally spherical and the outer surface (208) is contoured to mimic the natural geometry of a surface of a metatarsal bone.

4. The implant (200) of Claim 1, wherein the outer surface (208) is separated from the inner surface (206) by, at most, one millimeter of material.

5. The implant (200) of Claim 1, wherein a pair of raised ridges (220) are separated by a groove (222).

6. The implant (200) of Claim 1, wherein at least one of the plurality of raised ridges (220) includes at least one notch (232) adapted to prevent the stem (204) from backing out of a bone (102) once the stem (204) is implanted into the bone (102).

7. The implant (200) of Claim 6, wherein the at least one notch (232) is a hook-shaped recession in the at least one ridge (220).

8. The implant (200) of Claim 1, wherein the plurality of raised ridges traverse a majority of the portion (228) of the stem (202) overhung by the edge (210).

9. The implant (200) of Claim 1, wherein a height of at least one of the plurality of raised ridges (220) generally increases relative to a central axis (216) of the stem (202) as the at least one raised ridge (220) approaches the inner surface (206) of the cap (204).

10. The implant (200) of Claim 1, wherein the inner surface (206) of the cap (204) and the stem (202) include a porous coating and a biologic coating to promote bone growth.

11. The implant (200) of Claim 1, wherein at least one of the raised ridges (220) includes one or more notches (232) creating one or more teeth in the raised ridge (220).

## Patentansprüche

1. Ein Implantat (200) zur Verwendung an einem gelenkseitigen Ende eines Knochens bestehend aus:
einer Kappe (204) mit einer konvexen Außenfläche (208), die auf einer konkaven Innenfläche (206) aufliegt; und
einem Schaft (202), der aus der Innenfläche (206) hervorragt, so dass ein Rand (210) der Innenfläche (206) den Schaft (202) umgibt und über einen Teil des Schafts (202) bis zu Innenfläche (206) führend hervorsteht, wobei der Schaft (202) eine Mehrzahl von angehobenen Rippen (220) parallel zu seiner Länge aufweist, die nahe an der Spitze (224) des Schafts (202) beginnt und bis zu dem Teil des Schafts (202) verläuft, der von Rand (210) überragt wird, **dadurch gekennzeichnet, dass** der Schaft (202) einen glatten zylindrischen Abschnitt (230) zwischen der Spitze (224) und der Mehrzahl der angehobenen Rippen (220) aufweist.

2. Das Implantat (200) entsprechend Anspruch 1, wobei:
die Außenfläche (208) eine vertikale Achse (212) und eine horizontale Achse (214) aufweist, wobei die vertikale Achse (212) senkrecht zur horizontalen Achse (214) sitzt; und
ein Grad der Krümmung der Außenfläche (208) entlang der vertikalen Achse (212) größer als ein Grad der Krümmung der Außenfläche (208) entlang der horizontalen Achse (214) ist

3. Das Implantat (200) entsprechend Anspruch 1, wobei die Innenfläche (206) allgemein kugelförmig und die Außenfläche (208) so konturiert ist, dass sie die natürliche Geometrie der Oberfläche eines Mittelfußknochens nachahmt.

4. Das Implantat (200) entsprechend Anspruch 1, wobei die Außenfläche (208) von der Innenfläche (206) um maximal einen Millimeter an Material getrennt ist.

5. Das Implantat (200) entsprechend Anspruch 1, wobei ein Paar angehobener Rippen (220) durch eine Rille (222) getrennt wird.

6. Das Implantat (200) entsprechend Anspruch 1, wobei zumindest eine der Mehrzahl an angehobenen Rippen (220) zumindest eine Kerbe (232) aufweist, die so adaptiert ist, dass sie verhindert, dass der Schaft (204) rückwärts aus einem Knochen (102) gleitet, wenn der Schaft (204) in den Knochen (102) implantiert wurde.

7. Das Implantat (200) entsprechend Anspruch 6, wobei die zumindest eine Kerbe (232) eine hakenförmige Vertiefung in der zumindest einen angehobenen Rippe (220) ist.

8. Das Implantat (200) entsprechend Anspruch 1, wobei die Mehrzahl der angehobenen Rippen quer über einen Großteil des Abschnitts (228) des Schafts (20) verlaufen, der vom Rand (210) überragt wird.

9. Das Implantat (200) entsprechend Anspruch 1, wobei die Höhe von zumindest einer der Mehrzahl an angehobenen Rippen (220) allgemein relativ zur Mittelachse (216) des Schafts (202) zunimmt, wenn sich die zumindest eine angehobene Rippe (220) sich der Innenfläche (206) der Kappe (204) nähert.

10. Das Implantat (200) entsprechend Anspruch 1, wobei die Innenfläche (206) der Kappe (204) und des Schafts (202) eine poröse Beschichtung und eine biologische Beschichtung zur Förderung des Knochenwachstums aufweisen.

11. Das Implantat (200) entsprechend Anspruch 1, wobei die zumindest eine angehobene Rippe (220) eine oder mehrere Kerben (232) aufweist und so einen oder mehrere Zähne in der angehobenen Rippe (220) bildet.

## Revendications

1. Un implant (200) destiné à une utilisation sur une extrémité faisant face à une articulation d'un os comprenant :
un capuchon (204) possédant une surface extérieure convexe (208) recouvrant une surface intérieure concave (206), et
une tige (202) faisant saillie à partir de la surface intérieure (206) de sorte qu'un bord (210) de la surface intérieure (206) entoure la tige (202) et surplombe une partie (228) de la tige (202) conduisant à la surface intérieure (206), la tige (202) comprenant une pluralité d'arêtes en relief (220) parallèles à sa longueur qui débutent près d'une pointe (224) de la tige (202) et s'étendent dans la partie (228) de la tige (202) surplombée par le bord (210), **caractérisée en ce que** la tige (202) comprend une partie cylindrique lisse (230) disposée entre la pointe (224) et la pluralité d'arêtes en relief (220).

2. L'implant (200) selon la Revendication 1, où :
la surface extérieure (208) comprend un axe vertical (212) et un axe horizontal (214), l'axe vertical (212) étant situé perpendiculairement à l'axe horizontal (214), et
un degré de courbure de la surface extérieure (208) le long de l'axe vertical (212) est supérieur à un degré de courbure de la surface extérieure (208) le long de l'axe horizontal (214).

3. L'implant (200) selon la Revendication 1, où la surface intérieure (206) est généralement sphérique et la surface extérieure (208) est profilée de façon à imiter la géométrie naturelle d'une surface d'un os métatarsien.

4. L'implant (200) selon la Revendication 1, où la surface extérieure (208) est séparée de la surface intérieure (206) par, au maximum, un millimètre de matériau.

5. L'implant (200) selon la Revendication 1, où une paire d'arêtes en relief (220) sont séparées par une rainure (222).

6. L'implant (200) selon la Revendication 1, où au moins une arête de la pluralité d'arêtes en relief (220) comprend au moins une encoche (232) adaptée de façon à empêcher la tige (204) de ressortir d'un os (102) une fois que la tige (204) est implantée dans l'os (102).

7. L'implant (200) selon la Revendication 6, où la au moins une encoche (232) est un évidement en forme de crochet dans la au moins une arête (220).

8. L'implant (200) selon la Revendication 1, où la pluralité d'arêtes en relief traversent une majorité de la partie (228) de la tige (202) surplombée par le bord (210).

9. L'implant (200) selon la Revendication 1, où une hauteur d'au moins une arête de la pluralité d'arêtes en relief (220) augmente généralement par rapport à un axe central (216) de la tige (202) à mesure que la au moins une arête en relief (220) s'approche de la surface intérieure (206) du capuchon (204).

10. L'implant (200) selon la Revendication 1, où la surface intérieure (206) du capuchon (204) et la tige (202) comprennent un revêtement poreux et un revêtement biologique destinés à favoriser une croissance osseuse.

11. L'implant (200) selon la Revendication 1, où au moins une des arêtes en relief (220) comprend une ou plusieurs encoches (232) créant une ou plusieurs dents dans l'arête en relief (220).
